# EUROPEAN PATENT SPECIFICATION

(11) **EP 4 139 472 B1**
(45) Date of publication and mention of the grant of the patent: **23.09.2026**
(21) Application number: 21737203.6
(22) Date of filing: 22.04.2021
(51) Int. Cl.: C12Q 1/02, G01N 33/50, G01N 1/36

(54) **A METHOD FOR BIOTESTING OF A CONDENSED PHASE SUBSTANCES AND/OR MIXTURES ECOTOXICITY AND USE OF THE METHOD IN ECOTOXYCOLOGY**
VERFAHREN ZUR BIOPRÜFUNG VON STOFFEN IN KONDENSIERTER PHASE UND/ODER VON MISCHUNGEN, UMWELTFREUNDLICHE AUSRÜSTUNG UND VERWENDUNG DES VERFAHRENS
PROCÉDÉ D'ANALYSE BIOLOGIQUE DE L'ÉCOTOXICITÉ DE SUBSTANCES ET/OU DE MÉLANGES EN PHASE CONDENSÉE ET UTILISATION DU PROCÉDÉ EN ÉCOTOXICOLOGIE

(30) Priority: 22.04.2020 PL 43362320
(43) Date of publication of application: 01.03.2023
(73) Proprietor: Uniwersytet Jana Dlugosza w Czestochowie, 42-200 Czestochowa (PL); Centrum Badan Molekularnych I Makromolekularnych PAN, 90-363 Lodz (PL)
(72) Inventor: TUREK, Marika, 42-244 Mstow (PL); ROZYCKA-SOKOLOWSKA, Ewa, 42-200 Czestochowa (PL); BALCZEWSKI, Piotr, 91-225 Lodz (PL); KOPROWSKI, Marek, 91-117 Lodz (PL); OWSIANIK, Krzysztof, 95-200 Pabianice (PL)
(74) Representative: Malewska, Ewa
(86) International application number: PCT/PL2021/000025
(87) International publication number: WO 2021/215944

(56) References cited:
- TUREK MARIKA ET AL: "Modification of the Microtox Basic Solid Phase Test: A new application for the ecotoxicological studies on poorly soluble antihypertensive drugs", JOURNAL OF HAZARDOUS MATERIALS, vol. 399, 19 May 2020 (2020-05-19), AMSTERDAM, NL, pages 122839, XP055836197, ISSN: 0304-3894, DOI: 10.1016/j.jhazmat.2020.122839
- BICZAK R ET AL: "An influence of structural changes in ammonium cations on ecotoxicity of 2,2'-thiodiacetate mono and bis-salts", ECOTOXICOLOGY AND ENVIRONMENTAL SAFETY, ACADEMIC PRESS, SAN DIEGO, CA, US, vol. 155, 28 February 2018 (2018-02-28), pages 37 - 42, XP085363607, ISSN: 0147-6513, DOI: 10.1016/J.ECOENV.2018.02.062
- ACHESON CAROLYN M. ET AL: "COMPARING THE SOLID PHASE AND SALINE EXTRACT MICROTOX ASSAYS FOR TWO POLYCYCLIC AROMATIC HYDROCARBON-CONTAMINATED SOILS", ENVIRONMENTAL TOXICOLOGY AND CHEMISTRY, vol. 23, no. 2, 1 January 2004 (2004-01-01), US, pages 245 - 251, XP055836203, ISSN: 0730-7268, DOI: 10.1897/02-618
- HARKEY GAIL A. ET AL: "Effect of soil contaminant extraction method in determining toxicity using the microtox assay", ENVIRONMENTAL TOXICOLOGY AND CHEMISTRY, vol. 19, no. 2, 1 February 2000 (2000-02-01), US, pages 276 - 282, XP055836207, ISSN: 0730-7268, DOI: 10.1002/etc.5620190205
- TUREK MARIKA ET AL: "Ecotoxicity of ammonium chlorophenoxyacetate derivatives towards aquatic organisms: Unexpected enhanced toxicity upon oxygen by sulfur replacement", JOURNAL OF HAZARDOUS MATERIALS, ELSEVIER, AMSTERDAM, NL, vol. 382, 22 August 2019 (2019-08-22), XP085883734, ISSN: 0304-3894, [retrieved on 20190822], DOI: 10.1016/J.JHAZMAT.2019.121086

## Description

The invention relates to a method for biotesting of ecotoxicity of substances and mixtures in the condensed phase - solid and liquid, making use of bioluminescent bacteria, and application of the method in ecotoxicology. The invention allows for testing a wide variety of substances and mixtures, including poorly water-soluble and hydrophobic substances, among others drugs.

A rapid economic and urban development observed in the recent years carries the risk of environmental contamination, including contamination of water in natural reservoirs, artificial reservoirs and waterworks. To ensure ecological and human safety, it is necessary to monitor regularly ecotoxicity of waters, soils, bottom sediments, and also other solid substances that may affect well-being of organisms living in the water reservoirs and organisms that use the water.

The Microtox^{®} test, i.e. a method of testing the acute and chronic ecotoxicity of water, sewage and chemical compounds using bioluminescent bacteria *Aliivibrio fischeri* (before *2007: Vibrio fischeri),* has been known since 1979 [1]. It involves measurement of the disappearance of the *A. fischeri* bacteria bioluminescence, when exposed to the toxic effect of a sample tested. As a result of the test, the EC₅₀ value is obtained, i.e. the effective concentration of the substance tested causing 50% inhibition of bioluminescence in the bacterial population. Importantly, *A. fischeri* bacteria show a correlation of toxicity with higher organisms, such as: fish, duckweed, crustaceans or algae [2], which make the results of the Microtox^{®} ecotoxicity test conclusive and reliable, and thus the test itself readily used. Typically, the Microtox^{®} test is used in its basic form, involving the use of aqueous solutions of the tested compounds, which are mixed directly and in an appropriate dilution with a reagent containing bioluminescent bacteria *A. fischeri* (the bacterial reagent is easily produced in laboratories using biological material supplied in a freeze-dried form). Then, a bioluminescence of the thus prepared mixture is tested. Unfortunately, such a procedure is not applicable to poorly water-soluble or hydrophobic materials, including chemical compounds and mixtures thereof.

Three commercially available solid phase ecotoxicity tests are known: Microtox^{®} Basic Solid Phase Test, Toxi-Chromo Test^{™} and Ostracodtoxkit F^{™} test. Details of these tests are presented below and their most important features are summarized below, in Table 1.

**Table 1. The known commercial solid phase ecotoxicity tests utilizing direct contact of the test organism with the tested sample: Microtox^{®}, Toxi-Chromo Test^{™}, Ostracodtoxkit F^{™}.**

| **Test** | **Toxicity correlation with higher organisms** | **Duration** | **Endpoint** | **Applicability Examples** |
|---|---|---|---|---|
| Microtox Basic Solid Phase Test (*A. fischeri*) | correlation with benthic zone invertebrates (*C. riparus, H. Azteca, Hexagenia spp., T. tubifex*) | 1h | inhibition of bioluminescence after 5-30 minutes (EC₅₀) | soils contaminated with polycyclic aromatic hydrocarbons, bottom sediments, remediation sediments, residues from water treatment |
| Toxi-Chromo Test^{™} *(E. coli)* | correlation with benthic zone invertebrates *(C. riparus, H. Azteca, Hexagenia spp., T. tubifex*); Poor correlation with *Daphnia magna* | 2,5 h | decrease in absorbance after 90 minutes (EC₅₀) | bottom sediments, carbamazepine and its degradation products |
| Ostracodtoxkit F^{™} (*H. incongruens*) | correlation only with other crustaceans (*Hyalella azteca*) and insects (*Chironomus riparius* larvae) | 8 days | growth inhibition after 6 days (EC₅₀); mortality after 6 days (LC₅₀) | ionic liquids, sewage, sewage sludge, soils |

There is a commercially used solid-phase variant of the Microtox^{®} test, which was developed to assess the toxicity of soils and bottom sediments [3,4,5]. The measurement procedure involves pouring a standardized amount of solid (7 g) into 35 mL of a diluent solution (Microtox^{®} Solid Phase Diluent: 3.5% aqueous NaCl solution), and then vigorously mixing the suspension to homogenize the suspension properly, and then collect a representative sample (2 mL) from the center of the vortex. The sample collected in this way is then subjected to a series of dilutions, and the ecotoxicity of each of the obtained solutions is tested against *A. fischeri,* similarly as in the classic Microtox^{®} test. The Microtox^{®} test variant presented here is faster and more sensitive than other commercial solid-phase tests (Toxi-Chromo Test^{™}, Ostracodtoxkit F^{™}) and takes just 1 hour to complete testing. Please note that this original procedure has been developed to test the toxicity of soils and bottom sediments - solid mixtures of relatively low toxicity and low solubility. This method is not suited to the testing of poorly soluble or hydrophobic chemicals and mixtures, especially those with high toxicity, for example drugs, whose effective high concentration could cause death of the A. *fischeri* bcteria. Moreover, the necessity of using of 7 g of the analyte excludes the possibility of routine application of this method to the toxicity testing of expensive substances and newly synthesized chemical compounds, which are usually obtained in much smaller amounts in laboratory tests.

The commercially available Toxi-Chromo Test^{™} [6] for testing toxicity of solids is known. The Toxi-Chromo Test^{™} provides colorimetric testing of a solution obtained by mixing a tested solid with a solution containing β-galactosidase-producing *Escherichia coli.* This test allows for the growth of bacteria in direct contact with the tested sample. It has been proven that the results obtained with the bacterial Toxi-Chromo Test^{™} and Microtox^{®} tests for solids are correlated [7]. Unfortunately, Toxi-Chromo Test^{™} has similar drawbacks to Microtox^{®} Basic Solid Phase Test, i.e. it requires relatively large amounts of sample and is only suitable for low toxic solids such as soils and bottom sediments. Moreover, Toxi-Chromo Test^{™} is less sensitive than Microtox^{®} test, and the duration time is 2.5 hours [8].

The commercially available Ostracodtoxkit F^{™} test is also known [9]. This test uses the crustaceans *Heterocypris incongruens* and ensures their direct contact with the tested solid sample. The test is intended primarily for testing the toxicity of bottom sediments. Unfortunately, the duration time of the test is as much as 8 days (including the time needed for the necessary incubation of cysts), which excludes the possibility of using this method for quick screening tests. Furthermore, the Ostracodtoxkit F^{™} test is inconvenient because the crustaceans used in the method as test organisms must be cultured in advance.

Several modifications of the non-commercial ecotoxicity tests of solids are known to ensure direct contact of the test organism with the tested solid sample. For example, recombinant bacteria *Nitrosomonas europaea* were adapted to study soil contamination *in situ* [10]. In addition, attempts have also been made to modify the tests originally using only liquid samples. Until now, such modifications have been introduced mainly to assess the toxicity of soils contaminated with heavy metals. For example, the study of soil contaminated with zinc and cadmium using *Daphnia magna* crustaceans led to the conclusion that suspended (undissolved) heavy metal particles are more toxic than dissolved ones [11]. *Daphnia magna* crustaceans have also been used to evaluate and identify toxic compounds associated with river sediments [12]. Similar studies were carried out on soils contaminated with diesel oil, where the organism *Dictyostelium discoideum* was used [13].

There is an unmet need to develop a cheap, sensitive and quick method for testing the ecotoxicity of poorly soluble or hydrophobic chemicals and mixtures, especially solid, which would allow the measurement to be carried out with a relatively small sample of the tested material.

### Summary of the invention.

A method for biotesting of ecotoxicity of substances and mixtures in the condensed phase, including preparation of a dispersion of a tested condensed sample with a diluent and subjecting it to an intensive mixing to form a stable vortex, sampling the dispersion while mixing and subjecting the taken portion to a series of dilutions, and then adding to each of thus prepared samples a portion of an *A. fischeri* bacterial reagent and subjecting the obtained mixtures to testing of the bioluminescence inhibition, according to the invention is characterized in that a synthetic sample containing the tested condensed substance or a mixture of the condensed substances and an auxiliary substance, being a filler or carrier for the substance or a mixture of the condensed substances to be tested is subjected to biotesting, after it has been dispersed in the diluent. According to the invention, the condensed substance or mixture of the condensed substances is in solid or liquid phase. The auxiliary substance is solid, inert in the reaction environment and towards bioluminescent bacteria substance, preferably sand, quartz sand (SiO₂), titanium dioxide (TiO₂), aluminum trioxide (Al₂O₃), mesoporous silica, clay materials (kaolin, metakaolin, bentonite, montmorillonite), celite, silica gel, graphite, carbon nitride and insoluble non-toxic inorganic oxides. According to the invention, the weight/volume ratio of the synthetic sample to the diluent ranges from 1:4 g/mL to 1:6 g/mL, preferably 1:5 g/mL. According to the invention, in the case where a pure substance or a mixture of pure substances is tested, the content of the tested substance or mixture of substances in the synthetic sample is 0.5-10%, preferably 0.5-2.5%. Alternatively, when the tested substance is a component of a formulation, solution or dispersion, the content of that formulation, solution or dispersion in the synthetic sample is 1-20%, preferably 2.5-14%.

The invention also covers the use of the method defined above in ecotoxicology to characterize harmful effects caused by chemicals, to assess the environmental risk of micropolutants and their impact on ecosystem processes, and to design substances that are safer for the environment. According to the invention, ecotoxicity testing is carried out at the stage of planning, production and quality control of products in the pharmaceutical, cosmetic, food and chemical industries, which products, as a result of normal usage, may get into water reservoirs and affect living organisms in their natural environment.

The present invention ensures maximum direct contact of the tested sample (solid or liquid) with the test organism (i.e. bioluminescent bacteria) and determination of the toxicity of both dissolved, suspended and undissolved fractions of the tested chemical substance, which may be present in a phase other than the aqueous phase (sediment, liquid phase immiscible with water). This reflects the real situation in the natural environment, where organisms in water are exposed to a variety of chemical compounds that can be dissolved, suspended and/or adsorbed on a solid matrix, and not only dissolved in the water phase or found in soil and sediments. The present invention provides a possibility of testing the ecotoxicity of pure substances, as well as mixtures, solutions, dispersions and formulations, e.g. pharmaceutical, cosmetic, food and chemical.

The method for biotesting of ecotoxicity of the condensed phase substances and mixtures and the use of the method in the ecotoxicology according to the invention are described below in detail in the examples, with reference to the attached figures, in which:

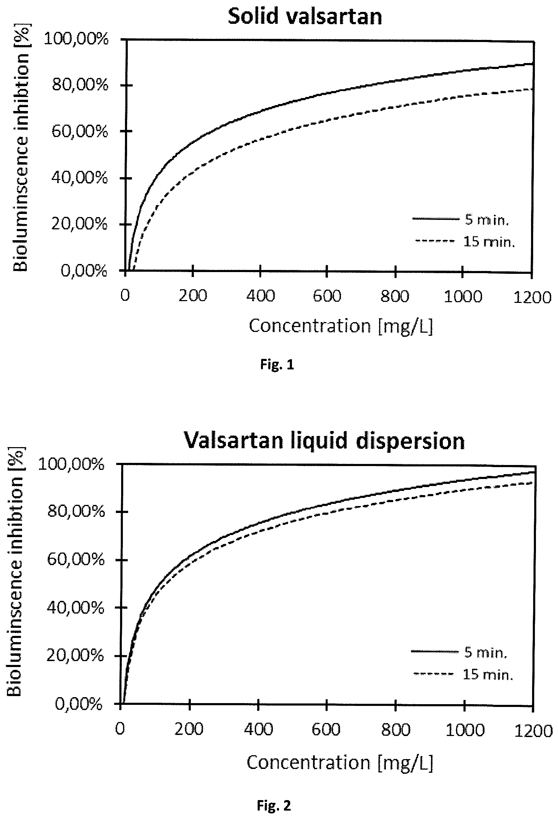

- Fig. 1: shows the bioluminescence inhibition curves for *A. fischeri* bacteria exposed to pure valsartan according to Example 1, with concentration of valsartan increasing within the range of 0-1200 mg/L, recorded for the exposure times of 5 and 15 minutes (averaged curves of three measurements).
- Fig. 2: shows the bioluminescence inhibition curves for *A. fischeri* bacteria exposed to pure valsartan in liquid form according to Example 2, with valsartan concentration increasing within the range 0-1200 mg/L, recorded for the exposure times of 5 and 15 minutes (curves averaged over three measurements).

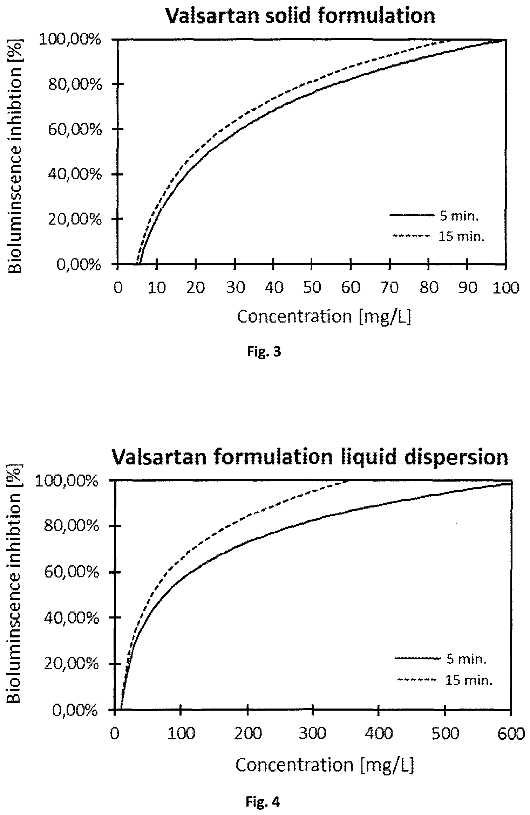

- Fig. 3: shows the bioluminescence inhibition curves for *A. fischeri* bacteria exposed to the valsartan formulation according to Example 3, with concentration of valsartan increasing within the range 0-100 mg/L, recorded for the exposure times of 5 and 15 minutes (averaged curves of three measurements).
- Fig. 4: shows the bioluminescence inhibition curves for *A. fischeri* bacteria exposed to the liquid formulation of valsartan according to Example 4, with concentration of valsartan increasing within the range of 0-600 mg/L, recorded for the exposure times of 5 and 15 minutes (curves averaged over three measurements).

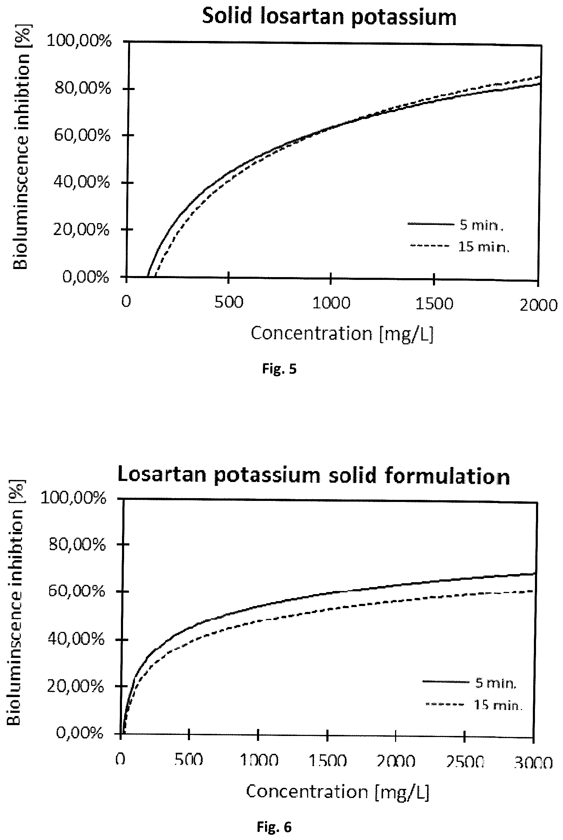

- Fig. 5: shows the bioluminescence inhibition curves for *A. fischeri* bacteria exposed to pure losartan potassium according to Example 5, with concentration of losartan potassium salt increasing within the range 0-2000 mg/L, recorded for the exposure times 5 and 15 minutes (curves averaged from three measurements).
- Fig. 6: shows the bioluminescence inhibition curves for *A. fischeri* bacteria exposed to the formulation of losartan potassium salt according to Example 6, with concentration of losartan potassium salt increasing within the range 0-3000 mg/L, recorded for the exposure times of 5 and 15 minutes (curves averaged from three measurements).

### Detailed description of the invention.

The present invention provides a possibility of testing the ecotoxicity of poorly soluble and hydrophobic condensed substances and mixtures (solid or liquid), including materials showing significant toxicity towards *A. fischeri* bioluminescent bacteria.

According to the invention, the method for measuring the ecotoxicity of condensed substances and mixtures is a modification of the Microtox^{®} Basic Solid Phase Test, which is the best commercially available test of this type known in the state of art. In principle, the present method of measurement, sampling and principle of operation is identical to the methodology of the Microtox^{®} Basic Solid Phase Test, and the key difference increasing its versatility and applicability in comparison to the Microtox^{®} Test lies in the method of preparation a solid sample for ecotoxicity testing. The described modification allows for a toxicity assessment of poorly soluble and hydrophobic substances that cannot be tested in the form of aqueous solutions due to poor solubility or immiscibility with water.

According to the invention, instead of a homogeneous solid sample with a relatively large mass (7 g in the Microtox^{®} Basic Solid Phase Test), which is often unreachable for laboratory measurements of expensive or innovative materials, a so-called synthetic sample (as opposed to natural samples, i.e. soils and bottom sediments) of the same mass, containing the tested substance and an inert auxiliary substance (so called solid matrix) is being created. As a result, the synthetic sample simulates the soil contaminated with the tested substance. The synthetic sample prepared in such a way can be dispersed in the diluent and processed further according to the Microtox^{®} Basic Solid Phase Test protocol. The Microtox^{®} Basic Solid Phase Test [5] protocol provides a series of steps covering, in turn:
- suspending 7 g of the test sample in 35 ml of diluent in a 50 ml beaker, mixing for 10 min;
- taking 2 mL of the suspension described above, while stirring it, into an appropriate cuvette;
- performing a series of 1:2 dilutions of the test sample using the diluent;
- reconstitution of freeze-dried *Aliivibrio fischeri* bacteria;
- preparation and 15 min incubation of the reagent (10 µL of reconstituted bacteria and 0.5 mL of diluent);
- bioluminescence measurement for time zero (bioluminescence before contact with the sample);
- adding the reagent to each test sample and to the control cuvette;
- incubation for 5 min, measurement of bioluminescence after 5 min incubation of bacteria with the sample;
- incubation for another 10 min, measurement of bioluminescence after 15 min incubation of bacteria with the sample.
The Microtox^{®} protocol provides for the use of a 30-well incubator block maintaining a temperature of 15° C in the wells of the sample cuvettes and 5.5° C in the well of the bacterial suspension.

In case of solids, the auxiliary substance acts mainly as a filler, allowing the tested sample to be diluted appropriately, and at the same time to achieve the appropriate weight required in the Microtox^{®} Basic Solid Phase Test. In case of liquid samples, the auxiliary substance can also act as a carrier, which is important especially in the case of hydrophobic samples, which permanently separate during mixing with diluent, and thus do not form homogeneous mixture during sampling. The use of an auxiliary substance as a carrier allows for the adsorption of hydrophobic liquids on the surface of the carrier and their testing in a pseudo-solid form, which gives a chance to obtain a repeatable and reliable test results.

As an auxiliary substance, a solid substance is used which is chemically inert in the reaction medium and inert to *A. fischeri* bioluminescent bacteria, i.e. which does not enhance or inhibit their bioluminescence. According to the invention, for example, sand, quartz sand (SiO₂), titanium dioxide (TiO₂), aluminum trioxide (Al₂O₃), mesoporous silica, clay materials (kaolin, metakaolin, bentonite, montmorillonite), celite, silica gel, graphite, carbon nitride and non-toxic inorganic oxides etc. are used. The use of sand and quartz sand has the advantage that the composition of the synthetic sample according to the invention prepared with the sand is qualitatively similar to that of the soil or bottom sediment containing natural sand. However, it is possible to use any auxiliary substance that meets the basic criterion of inertness.

According to the invention, to prepare the synthetic sample for the ecotoxicity test, the substance to be tested is mixed with an inert auxiliary substance (preferably sand or quartz sand) in a specific mass ratio depending on the intensity of the biological response to the presence of the tested substance. The optimal amount of the tested substance in the synthetic sample (the so-called dilution of the tested substance) requires prior experimental determination and may vary depending on its type. Due to the specificity of the measurement, the content of the tested substance (e.g. drug) in the tested synthetic sample is 0.5-10%, preferably 0.5-2.5%. In the case where the tested pharmaceutical substance is in the form of a tablet (containing usually additional medically inactive fillers), the content of the mixture constituting the tablet in the synthetic sample should be higher and range from 1-20%, preferably 2.5-14%, due to the lower content of the active substance in the tablet in comparison with the pure active substance.

According to the invention, a sample containing the substance to be test and an auxiliary substance is subjected to the procedure provided for in the Microtox^{®} Basic Solid Phase Test. The pre-suspension is made by mixing the synthetic sample with a diluent (Microtox^{®} Solid Phase Diluent: 3.5% aqueous NaCl solution) in appropriate proportions provided for in the measurement protocol, for example 7 g of the sample is mixed with 35 mL of diluent (weight/volume ratio of the sample to diluent is 1:5 g/mL), but after validation it is possible to use dispersions of any proportion (preferably from 1:4 g/mL to 1:6 g/mL) and scale (preferably samples weighing 3-10 g depending on capacity of the equipment used). In the case of hydrophobic chemicals, it is allowed to suspend them in a mixture of methanol with a diluent (e.g. 4% methanol solution) to increase their solubility. Then, the suspension is stirred in a tall beaker on a stirrer so that, as a result of mixing, the vortex always has the same, repeatable height, equal to half the height of the liquid level. A representative sample for further dilutions is drawn with an automatic pipette while the suspension is stirred, from half the depth of the suspension according to the Microtox^{®} protocol. The further steps in the measurement (including but not limited to dilution, sampling, luminescence measurement, etc.) also follow the Microtox^{®} Basic Solid Phase Test Procedure.

The sample preparation procedure described in the present invention ensures the direct contact of the tested substances (solid or liquid) with the bioluminescent *A. fischeri* bacteria and determination of the toxicity of both the dissolved fraction, the suspended fraction and any undissolved fractions that may be present in a different phase than the water phase (sediment, liquid phase). Thus far, such approach has not been used to test the ecotoxicity of poorly soluble and hydrophobic condensed substances. This type of substances includes, for example, pharmaceuticals, the presence of which in the environment is a significant emerging problem, because such micro-pollutants, despite being present in small amounts, are able to effectively affect non-target organisms. By using the method according to the invention, it becomes possible to test the ecotoxicity of both the pure active substance and the entire pharmaceutical formulation in tablet form.

The method of determining the ecotoxicity of condensed samples, according to the invention, reflects the real situation in the natural environment, where living organisms in water reservoirs are exposed to various chemical compounds and mixtures that may be dissolved and/or suspended and/or adsorbed on a solid matrix, and not just dissolved in the water phase or found in soil and bottom sediments. The approach presented in the present invention focuses on ensuring the maximum possible direct contact of the tested sample with the test organism, which is a key aspect of the correct determination of its ecotoxicity [7,14]. Most solid phase tests are based on indirect analysis of extracts and eluates, where water and/or organic solvents are used to elute the test toxicants from the solid phase [15]. This "indirect" approach ignores the synergistic/antagonistic interactions of the test organisms with the solid fraction [10].

The method according to the invention allows for a direct contact of a solid sample with bioluminescent bacteria *A. fischeri* and determination of the total toxicity resulting from the dissolved and undissolved fractions of the test chemical, which can be in all 3 forms, i.e. in solution, suspension and sediment. The method according to the invention allows for measurements to be carried out at the same time as for the Microtox^{®} Basic Solid Phase Test, and allows for very large material savings due to the possibility of using small amounts of valuable substances (much less than 3-10 g per measurement) to test their toxicity.

Due to the possibility of reliable and reproducible determination of the effective toxicity of substances in the aquatic environment in various forms, the method according to the invention can be used in ecotoxicology, i.e. to characterize the harmful effects caused by chemicals, assess the environmental risk of given micropolutants and their impact on ecosystem processes, as well as in designing substances that are safer for the environment.

The method according to the invention can be used in industry (for example: pharmaceutical, cosmetic, food, chemical, etc. industries) at the stage of planning, production and quality control of new products that, as a result of normal usage, may get into water reservoirs and affect living organisms in their natural environment.

The present invention provides the possibility of testing the ecotoxicity of both pure active substances, as well as mixtures, solutions, dispersions and formulations, e.g. pharmaceutical, cosmetic, food and chemical. This allows for testing the ecotoxicity of specific ready-made preparations and products, regardless of their solubility in water, and not, as previously, only pure substances that are well-soluble. The present invention significantly expands the analytical capabilities, compared to the limited applicability of the Microtox^{®} Basic Solid Phase Test method.

Moreover, the Microtox^{®} test, as the only one of the commercial solid phase tests (Table 1), is standardized according to the EN ISO 11348-3 standard, and the toxicity results obtained for *A. fischeri* bacteria may be correlated with the toxicity towards higher aquatic organisms [2]. The advantage of the present test is also a short analysis time (approx. 1 hour), high sensitivity of the bacteria used compared to other bacterial tests, low cost of analysis and a reduced size of the sample needed for the measurement. Microtox^{®} Basic Solid Phase Test is widely used in ecotoxicity studies. To date (April 2020), 1,755 scientific articles about the Microtox^{®} test have been published, according to the Scopus database. For comparison, the number of scientific articles on the Ostracodtoxkit F^{™} test is 41, and the Toxi-Chromo Test^{™} just 17.

The method for biotesting of ecotoxicity of the condensed substances and mixtures and use of the method in ecotoxicology have been described in the working examples with reference to the tables and the drawings.

**Example 1. (valsartan ecotoxicity test)** 0.160 g of pure valsartan (drug, pure poorly soluble active ingredient) was mixed with 6.840 g of quartz sand to obtain the 7 g synthetic sample which was suspended in 35 mL Microtox^{®} Solid Phase Diluent in a 50 mL beaker, in a weight-by-volume ratio 1:5 g/L, and stirred on a magnetic stirrer for 10 minutes at 1000 rpm. A representative sample for further dilutions was taken from half the depth of the suspension without stirring interruption according to the Microtox^{®} Basic Solid Phase Test protocol. Further analysis was carried out according to the standard protocol foreseeing a serial dilution of the sample, measurement with a known bioluminescence inhibition method, and determination of the EC₅₀ value related to the concentration of the substance that resulted in a 50% inhibition of *A. fischeri* bioluminescence. The measurements were carried out in triplicate and the EC₅₀ values were determined after 5 and 15 minutes of exposure. The obtained results with 95% confidence intervals, as well as the correlation coefficients are summarized below, in Table 2.

**Table 2. EC₅₀ values [mg/L] obtained after 5 and 15 minutes exposure of A. fischeri bacteria to pure valsartan along with the corresponding confidence intervals.**

| **Replicate** | **EC_{50 5 min} [mg/L] (95% confidence interval)** | **EC_{50 15 min} [mg/L] (95% confidence interval)** | **Correlation factor (R²) 5 min; 15 min** |
|---|---|---|---|
| 1. | **169.24** (79.80-358.82) | **341.70** (338.33-358.97) | 0.8281; 0.9987 |
| 2. | **172.06** (102.02-290.17) | **242.80** (103.75-568.26) | 0.9527; 0.9927 |
| 3. | **88.31** (83.90-92.91) | **120.04** (99.20-145.28) | 0.9997; 0.9995 |
| **Mean:** | **143.20 ± 38.83** | **234.83 ± 90.66** | - |

**Example 2. (valsartan liquid dispersion ecotoxicity test)** *A. fischeri* bioluminescence inhibition measurements were performed as in Example 1, except that 6.840 g of quartz sand was mixed with 0.160 g of valsartan liquid dispersion in aqueous methanol (drug, dispersion of the poorly soluble active ingredient). Further operations were performed as in Example 1 according to the standard protocol of Microtox^{®} Basic Solid Phase Test. The obtained EC₅₀ results (after 5 and 15 minutes exposure of *A. fischeri* bacteria to the liquid dispersion of valsartan), the corresponding confidence intervals (95%) and correlation coefficients are summarized below, in Table 3.

**Table 3. EC₅₀ values [mg/L] obtained after 5 and 15 minutes exposure of A. fischeri bacteria to liquid dispersion of valsartan along with the corresponding confidence intervals.**

| **Replicate** | **EC_{50 5 min} [mg/L] (95% confidence interval)** | **EC_{50 15 min} [mg/L] (95% confidence interval)** | **Correlation factor (R²) 5 min; 15 min** |
|---|---|---|---|
| 1. | **84.08** (15.83-446.55) | **84.35** (19.19-370.77) | 0.9509; 0.9615 |
| 2. | **238.66** (102.09-557.85) | **202.56** (119.71-342.94) | 0.9493; 0.9236 |
| 3. | **74.63** (32.10-173.60) | **69.81** (28.30-172.31) | 0.8853; 0.8625 |
| **Mean:** | **132.46 ± 75.19** | **118.92 ± 59.46** | - |

**Example 3. (solid formulation of valsartan ecotoxicity test)** Bioluminescence inhibition measurements of *A. fischeri* bacteria were performed as in Example 1, except that 6.660 g of quartz sand was mixed with 0.340 g of VALTAP valsartan powder tablet (drug, commercially available poorly soluble pharmaceutical preparation), containing 0.160 g of pure valsartan and 0.180 g of excipients: microcrystalline cellulose, colloidal anhydrous silica, sorbitol, magnesium carbonate, pregelatinized maize starch, povidone, sodium stearyl fumarate, sodium lauryl sulfate, crospovidone. Further operations were performed as in Example 1 according to the standard protocol of Microtox^{®} Basic Solid Phase Test. The obtained EC₅₀ results (after 5 and 15 minutes exposure of *A. fischeri* bacteria to the solid formulation of valsartan), the corresponding confidence intervals (95%) and correlation coefficients are summarized below, in Table 4.

**Table 4. EC₅₀ values [mg/L] obtained after 5 and 15 minutes exposure of A. fischeri bacteria to the solid formulation of valsartan along with the corresponding confidence intervals.**

| **Replicate** | **EC_{50 5 min} [mg/L] (95% confidence interval)** | **EC_{50 15 min} [mg/L] (95% confidence interval)** | **Correlation factor (R²) 5 min; 15 min** |
|---|---|---|---|
| 1. | **41.30** (33.12-51.48) | **32.84** (27.05-39.88) | 0.9932; 0.9936 |
| 2. | **35.83** (16.67-70.73) | **28.06** (17.61-44.70) | 0.9265; 0.9610 |
| 3. | **62.12** (54.40-70.93) | **58.56** (50.89-67.27) | 0.9985; 0.9980 |
| **Mean:** | **45.42 ± 11.33** | **39.82 ± 13.39** | - |

**Example 4. (valsartan formulation liquid dispersion ecotoxicity test)** Bioluminescence inhibition measurements of *A. fischeri* bacteria were performed as in Example 1, except that 6.660 g of quartz sand was mixed with 0.340 g of VALTAP valsartan powder tablet, dispersed in an aqueous solution of methanol containing 0.160 g of pure valsartan and 0.180 g of excipients. Further operations were performed as in Example 1 according to the standard protocol of Microtox^{®} Basic Solid Phase Test. The obtained EC₅₀ results (after 5 and 15 minutes of exposure of *A. fischeri* bacteria to liquid valsartan dispersion), the corresponding confidence intervals (95%) and correlation coefficients are summarized below, in Table 5.

**Table 5. EC₅₀ values [mg/L] obtained after 5 and 15 minutes exposure of A. fischeri bacteria to the liquid dispersion of valsartan formulation along with the corresponding confidence intervals.**

| **Replicate** | **EC_{50 5 min} [mg/L] (95% confidence intervals)** | **EC_{50 15 min} [mg/L] (95% confidence intervals)** | **Correlation factor (R²) 5 min; 15 min** |
|---|---|---|---|
| 1. | **76.84** (47.80-123.33) | **58.59** (39.23-87.45) | 0.9692; 0.9723 |
| 2. | **65.69** (51.64-83.56) | **42.73** (30.67-59.56) | 0.9950; 0.9887 |
| 3. | **96.10** (54.38-169.84) | **75.49** (58.18-97.97) | 0.9809; 0.9859 |
| **Mean:** | **79.54 ± 12.56** | **58.93 ± 13.37** | - |

**Example 5. (losartan potassium ecotoxicity test)** *A. fischeri* bioluminescence inhibition measurements were carried out as in Example 1, except that 0.200 g of losartan potassium (drug, pure poorly soluble active ingredient) were mixed with 5.600 g of sand quartz to give the synthetic sample weighing 5.80 g, which was suspended in 35 mL of Microtox^{®} Solid Phase Diluent (ratio 1:6 g/mL). Further operations were performed as in Example 1 according to the standard protocol of Microtox^{®} Basic Solid Phase Test. The obtained EC₅₀ results (after 5 and 15 minutes of *A. fischeri* exposure to pure potassium salt of losartan), the corresponding confidence intervals (95%) and correlation coefficients are summarized below, in Table 6.

**Table 6. EC₅₀ values [mg/L] obtained after 5 and 15 minutes exposure of A. fischeri bacteria to the pure losartan potassium along with the corresponding confidence intervals.**

| **Replicate** | **EC_{50 5 min} [mg/L] (95% confidence interval)** | **EC_{50 15 min} [mg/L] (95% confidence interval)** | **Correlation factor (R²) 5 min; 15 min** |
|---|---|---|---|
| 1. | **490.17** (307.45-780.93) | **653.65** (319.89-1335.30) | 0.9196; 0.9188 |
| 2. | **594.25** (343.09-1028.98) | **694.10** (541.64-889.82) | 0.9101; 0.9906 |
| 3. | **667.23** (490.17-908.21) | **629.33** (172.93-2289.90) | 0.9858; 0,.691 |
| **Mean:** | **583.88 ± 72.65** | **659.02 ± 26.71** | - |

**Example 6. (losartan potassium solid formulation ecotoxicity test)** Bioluminescence inhibition of *A. fischeri* bacteria were performed as in Example 1, except that 7.910 g of quartz sand was mixed with 0.840 g of powdered tablets of LOZAP potassium salt (drug, commercially available poorly soluble pharmaceutical preparation), containing 0.200 g of pure potassium salt of losartan and 0.640 g of excipients: microcrystalline cellulose, mannitol, crospovidone, colloidal anhydrous silica, talc and magnesium stearate. The synthetic sample of 8.75 g was obtained, which was suspended in 35 mL of Microtox^{®} Solid Phase Diluent (ratio 1:4 g/mL). Further operations were performed as in Example 1 according to the standard protocol of Microtox^{®} Basic Solid Phase Test. The obtained EC₅₀ results (after 5 and 15 minutes of exposure of *A. fischeri* bacteria to the solid formulation of losartan potassium salt), the corresponding confidence intervals (95%) and correlation coefficients are summarized below, in Table 7.

**Table 7. EC₅₀ values [mg/L] obtained after 5 and 15 minutes exposure of A. fischeri bacteria to the solid formulation of losartan potassium along with the corresponding confidence intervals.**

| **Replicate** | **EC_{50 5 min} [mg/L] (95% confidence interval)** | **EC_{50 15 min} [mg/L] (95% confidence interval)** | **Correlation factor (R²) 5 min; 15 min** |
|---|---|---|---|
| 1. | **939.60** (524.11-1684.61) | **1200.95** (711.35-2026.57) | 0.9391; 0.9535 |
| 2. | **484.79** (220.39-1066.60) | **612.64** (222.60-1685.46) | 0.8247; 0.7855 |
| 3. | **603.02** (396.55-916.69) | **809.21** (528.07-1239.42) | 0.9536; 0.9450 |
| **Mean:** | **675.81 ± 192.68** | **874.27 ± 244.54** | - |

**Example 7. (alternative auxiliary substances)** The bioluminescence inhibition measurements of *A. fischeri* bacteria were performed analogously to Examples 1-6, except that natural sand, titanium dioxide and aluminum trioxide were used instead of quartz sand. The obtained results were consistent with the results presented above, in Tables 2-7.

### Cited literature

[01] Bulich, A.A., Use of Luminescent Bateria for Determining Toxicity in Aquatic Environments, w: Aquatic Toxicology, Marking L.L., Kimerle R.A. (eds), American Society for Testing and Materials STP, 1979, 667, 98-106.
[02] Kaiser, K.L., Correlations of Vibrio fischeri bacteria test data with bioassay data for other organisms. Environ. Health Perspect., 1998, 106(S2), 583-591.
[03] Beg, K.R., Shakir A.. Microtox Toxicity assay for the sediment quality assessment of Ganga River. Am. J. Environ. Sci., 2008, 4(4), 383-387.
[04] Doe, K., Jackman, P., Scroggins, R., McLeay, D., Wohlgeschaffen G. Solid-Phase Test for sediment toxicity using the luminescent bacterium, Vibrio Fischeri. In: Blaise C., Férard J.F. (eds) Small-scale freshwater toxicity investigations. Springer, 2008, Dordrecht.
[05] Microtox^{®} M500, ModernWater Inc, New Castle, United States. www.modernwater.com
[06] Toxi-Chromo Test^{™}, Environmental Bio-Detection Products Inc, Canada
[07] Kwan, K.K., Dutka, B.J., Comparative assessment of two solid-phase toxicity bioassays: The direct sediment toxicity testing procedure (DSTTP) and the Microtox® solid-phase test (SPT), Bull. Environ. Contam. Toxicol., 1995, 55(3), 338-346.
[08] Koopman, B., Bitton, G., Dutton, R.J., Logue, C.L., Toxicity testing in wastewater systems: Application of a short-term assay based on induction of the lac operon in E. coli, Water Sci. Techn., 1988, 20(137).
[09] Ostracodtoxkit F^{™}, MicroBioTests, Belgium. www.microbiotests.com
[10] Brandt, K.K., Pedersen, A., Sørensen, J., Solid-phase contact assay that uses a lux-marked Nitrosomonas europaea reporter strain to estimate toxicity of bioavailable linear alkylbenzene sulfonate in soil, Appl. Environ. Microbiol., 2002, 68(7) 3502-3508.
[11] Weltens, R., Goossens, R., Van Puymbroeck, S., Ecotoxicity of contaminated suspended solids for filter feeders (Daphnia magna), Arch. Environ. Contam. Toxicol., 2000, 39(3), 315-323.
[12] Rivetti, C., Gómez-Canela, C., Lacorte, S., Díez, S., Lázaro, W.L., Barata, C., Identification of compounds bound to suspended solids causing sub-lethal toxic effects in Daphnia magna. A field study on re-suspended particles during river floods in Ebro River, Aquatic Toxicol., 2015, 161, 41-50.
[13] Rodriguez-Ruiz, A., Dondero, F., Viarengo, A. and Marigómez, I., Toxicity assessment of diesel- and metal-contaminated soils through elutriate and solid phase assays with the slime mold Dictyostelium discoideum. Environ. Toxicol. Chem., 2016, 35(6), 1413-1421.
[14] Bitton, G., Garland, E., Kong, I.-Ch., Morel, J.L., Koopman, B., A direct solid-phase assay specific for heavy metal toxicity. I. methodology, J. Soil Contam., 1996, 5(4), 385-394.
[15] Shoji, R., Nakayama, H., Sakai, Y., Mohri, S., Yamada, M., Evaluation of the ecotoxicity of solid wastes using rapid leaching test and bioassays, J. Environ. Sci. Health A, 2008, 43(9), 1048-1053.

Research work on the present method for biotesting of ecotoxicity of condensed substances and mixtures and the use of the method in ecotoxicology was financed by the NCN Preludium project no. UMO-2019/33/N/ST5/01602 entitled "Co-crystallization and co-amorphization of the angiotensin II receptor antagonists leading to more soluble compounds of a bifunctional character".

## Claims

1. A method for biotesting of ecotoxicity of condensed substances and/or mixtures, including preparation of a dispersion of the tested sample with a diluent and subjecting it to intensive mixing to create a stable vortex, sampling the dispersion while mixing and subjecting the taken portion to a series of dilutions, and then adding to each of thus prepared samples a portion of an *A. fischeri* bacterial reagent and subjecting the obtained mixtures to testing of the bioluminescence inhibition, **characterized in that** a synthetic sample containing the tested condensed substance or a mixture of the condensed substances and an auxiliary substance, being a filler or carrier for the condensed substance or a mixture of condensed substances to be tested is subjected to biotesting, after it has been dispersed in the diluent.

2. The method according to claim 1, **characterized in that** the condensed substance or the mixture of the condensed substances to be tested is in solid or liquid phase.

3. The method according to claim 1, **characterized in that** the auxiliary substance is a solid, inert in the reaction medium and towards bioluminescent bacteria substance, preferably sand, quartz sand (SiO₂), titanium dioxide (TiO₂), aluminum trioxide (Al₂O₃), mesoporous silica, clay materials (kaolin, metakaolin, bentonite, montmorillonite), celite, silica gel, graphite, carbon nitride and insoluble non-toxic inorganic oxides.

4. The method according to claim 1, **characterized in that** in the case where the pure condensed substance or the mixture of pure condensed substances is tested, its content in the synthetic sample is 0.5-10%, preferably 0.5-2.5%.

5. The method according to claim 1, **characterized in that** in the case where the tested substance constitutes a component of a formulation, solution or dispersion, the content of said formulation, solution or dispersion in the synthetic sample is 1-20%, preferably 2.5-14%.

6. The method according to claim 1, **characterized in that** the weight/volume ratio of the synthetic sample to the diluent ranges from 1:4 g/mL to 1:6 g/mL, preferably 1:5 g/mL.

7. The use of the method as defined in claims 1-6 in ecotoxicology to characterize the harmful effects caused by chemicals, to assess the environmental risk of micropolutants and their impact on ecosystem processes, and to design substances that are safer for the environment.

8. Use according to claim 7, **characterized in that** the ecotoxicity test is carried out at the stage of planning, production and quality control of products in the pharmaceutical, cosmetic, food and chemical industries, which products, as a result of normal usage, may get into water reservoirs and affect living organisms in their natural environment.

## Patentansprüche

1. Verfahren zur Biotestung der Ökotoxizität von kondensierten Stoffen und/oder Gemischen, umfassend die Herstellung einer Dispersion der Testprobe mit einem Verdünnungsmittel und deren intensives Mischen zur Bildung eines stabilen Wirbels, die Entnahme von Proben aus der Dispersion während des Mischens und die anschließende Verdünnung der gesammelten Probe, die Zugabe eines Aliquots des *A. fischeri*-Bakterienreagenz zu jeder der so hergestellten Proben und die Durchführung eines Biolumineszenz-Abklingtests der resultierenden Gemische, **dadurch gekennzeichnet dass** der Biotest an einer synthetischen Probe durchgeführt wird, die den zu testenden kondensierten Stoff oder das Gemisch kondensierter Stoffe sowie einen festen Hilfsstoff enthält, der als Füllstoff oder Träger für den zu testenden kondensierten Stoff oder das Gemisch kondensierter Stoffe dient, nachdem dieser im Verdünnungsmittel dispergiert wurde.

2. Verfahren nach Anspruch 1, **dadurch gekennzeichnet dass** der zu testende kondensierte Stoff oder das Gemisch kondensierter Stoffe in fester oder flüssiger Phase vorliegt.

3. Verfahren nach Anspruch 1, **dadurch gekennzeichnet dass** es sich bei dem Hilfsstoff um einen festen Stoff handelt, der im Reaktionsmedium und gegenüber biolumineszenten Bakterien inert ist, vorzugsweise es sich um Sand, Quarzsand (SiO₂), Titandioxid (TiO₂), Aluminiumtrioxid (Al₂O₃), mesoporöses Siliciumdioxid, Tonminerale (Kaolin, Metakaolin, Bentonit, Montmorillonit), Celite, Kieselgel, Graphit, Kohlenstoffnitrid und unlösliche, ungiftige anorganische Oxide handelt.

4. Verfahren nach Anspruch 1, **dadurch gekennzeichnet dass** falls ein reiner kondensierten Stoff oder ein Gemisch reiner kondensierter Stoffe geprüft wird, denen Gehalt in der synthetischen Probe 0,5-10 %, vorzugsweise 0,5-2,5 %, beträgt.

5. Verfahren nach Anspruch 1, **dadurch gekennzeichnet dass** falls ein Stoff als Bestandteil einer Formulierung, Lösung oder Dispersion geprüft wird, der Gehalt der Formulierung, Lösung oder Dispersion in der synthetischen Probe 1-20 %, vorzugsweise 2,5-14 %, beträgt.

6. Verfahren nach Anspruch 1, **dadurch gekennzeichnet dass** das Massen-Volumen-Verhältnis der synthetischen Probe zum Verdünnungsmittel zwischen 1:4 g/ml und 1:6 g/ml, vorzugsweise 1:5 g/ml, liegt.

7. Anwendung des in den Ansprüchen 1 bis 6 definierten Verfahrens in der Ökotoxikologie zur Charakterisierung der schädlichen Wirkungen chemischer Substanzen, zur Bewertung des Umweltrisikos von Mikroverunreinigungen und deren Auswirkungen auf Ökosystemprozesse sowie zur Entwicklung umweltverträglicherer Substanzen.

8. Anwendung nach Anspruch 7, **dadurch gekennzeichnet dass** der Ökotoxizitätstest in der Planungs- , Produktions- und Qualitätskontrollphase von Produkten der pharmazeutischen, kosmetischen, Lebensmittel- und chemischen Industrie durchgeführt wird, die im Rahmen ihrer normalen Verwendung in Gewässer gelangen und Lebewesen in ihrem natürlichen Lebensraum beeinträchtigen können.

## Revendications

1. Procédé de biotest de l'écotoxicité de substances condensées et/ou de mélanges, comprenant les étapes suivantes : préparation d'une dispersion de l'échantillon à tester avec un diluant et agitation intensive de cette dispersion afin de former un vortex stable ; prélèvement d'échantillons de la dispersion pendant l'agitation ; dilution subséquente de l'échantillon prélevé ; ajout d'une aliquote du réactif bactérien *A. fischeri* à chacun des échantillons ainsi préparés ; et réalisation d'un test de décroissance de la bioluminescence des mélanges obtenus, **caractérisé en ce que** le biotest est réalisé sur un échantillon synthétique contenant la substance condensée ou le mélange de substances condensées à tester, ainsi qu'une substance auxiliaire solide servant de charge ou de support à la substance condensée ou au mélange de substances condensées à tester après dispersion dans le diluant.

2. Procédé selon la revendication 1, **caractérisé en ce que** la substance condensée ou le mélange de substances condensées à tester se trouve à l'état solide ou liquide.

3. Procédé selon la revendication 1, **caractérisé en ce que** la substance auxiliaire est un solide inerte dans le milieu réactionnel et vis-à-vis des bactéries bioluminescentes, de préférence du sable, du sable de quartz (SiO₂), du dioxyde de titane (TiO₂), du trioxyde d'aluminium (Al₂O₃), du dioxyde de silicium mésoporeux, des minéraux argileux (kaolin, métakaolin, bentonite, montmorillonite), de la célite, du gel de silice, du graphite, du nitrure de carbone et des oxydes inorganiques insolubles et non toxiques.

4. Procédé selon la revendication 1, **caractérisé en ce que** au cas où la substance condensée pure ou en mélange de substances condensées pures dans l'échantillon synthétique est testée, la concentration pertinente est de 0,5 à 10 %, de préférence de 0,5 à 2,5 %.

5. Procédé selon la revendication 1, **caractérisé en ce que** au cas où la substance comme composant d'une formulation, d'une solution ou d'une dispersion est testée, la concentration de la formulation, de la solution ou de la dispersion dans l'échantillon synthétique est de 1 à 20 %, de préférence de 2,5 à 14 %.

6. Procédé selon la revendication 1, **caractérisé en ce que** le rapport massique/volumique de l'échantillon synthétique au diluant est compris entre 1:4 g/ml et 1:6 g/ml, de préférence 1:5 g/ml.

7. Application du procédé défini dans les revendications 1 à 6 en écotoxicologie pour la caractérisation des effets nocifs des substances chimiques, l'évaluation du risque environnemental des micropolluants et de leur impact sur les processus écosystémiques, ainsi que pour le développement de substances plus respectueuses de l'environnement.

8. Application selon la revendication 7, **caractérisée en ce que** le test d'écotoxicité est réalisé lors des phases de planification, de production et de contrôle qualité des produits des industries pharmaceutique, cosmétique, alimentaire et chimique qui, au cours de leur utilisation normale, peuvent se retrouver dans les cours d'eau et nuire aux êtres vivants dans leur milieu naturel.
